# EUROPEAN PATENT APPLICATION

(11) **EP 4 840 860 A1**
(43) Date of publication of application: **18.11.2026**
(21) Application number: 25738539.3
(22) Date of filing: 10.01.2025
(51) Int. Cl.: C07D 487/14, A61K 31/4985

(54) **SYNTHESIS PROCESS FOR UPADACITINIB OR HYDRATE THEREOF**

(30) Priority: 11.01.2024 CN 202410041809
(71) Applicant: Zhejiang Huahai Pharmaceutical Co., Ltd., Taizhou, Zhejiang 317024 (CN)
(72) Inventor: WANG, Pingzhong, Taizhou, Zhejiang 317024 (CN); YANG, Zhengyi, Taizhou, Zhejiang 317024 (CN); WANG, Yao, Taizhou, Zhejiang 317024 (CN); GU, Hong, Taizhou, Zhejiang 317024 (CN); SU, Hu, Taizhou, Zhejiang 317024 (CN); HUANG, Luning, Taizhou, Zhejiang 317024 (CN)
(74) Representative: Noble, Frederick
(86) International application number: PCT/CN2025/071650
(87) International publication number: WO 2025/149011

(57) **Abstract**

Provided is a synthesis process for Upadacitinib or a hydrate thereof, relating to the field of drug synthesis. The synthesis process specifically comprises: reacting a compound of formula (2) and a compound of formula (3) in an organic solvent and water, and upon completion of the reaction, mixing a reaction system with inorganic alkali A for reaction to obtain a compound of formula (1) or a hydrate thereof. The method involves simple and convenient operations, the product is easy to obtain, the product has high purity, and amplified production and large-scale pro-duction can be stably realized.

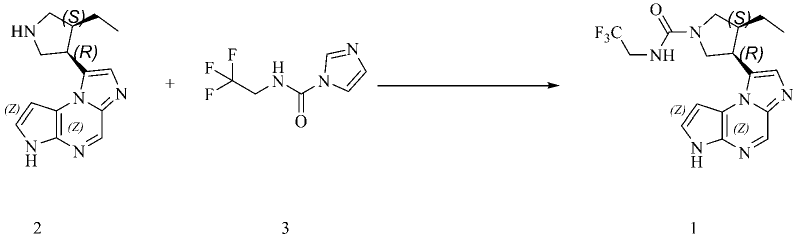

## Description

### CROSS-REFERENCE TO RELATED APPLICATION

The present application claims priority to and benefit of Chinese Patent Application No. 202410041809.2, filed with the China National Intellectual Property Administration, which is incorporated herein by reference in its entirety.

### TECHNICAL FIELD

The present application belongs to the field of drug synthesis, and specifically relates to a synthesis process for Upadacitinib or a hydrate thereof.

### BACKGROUND

Upadacitinib is a novel oral selective Janus kinase (JAK) inhibitor developed by AbbVie Inc. of the United States. It was approved for marketing by the U.S. Food and Drug Administration (FDA) in August 2019. The marketed product is a hemihydrate crystal with the trade name Rinvoq, and is used for the treatment of rheumatoid arthritis, psoriatic arthritis, atopic dermatitis, ulcerative colitis, Crohn's disease, ankylosing spondylitis, and non-radiographic axial spondyloarthritis.

The chemical name of Upadacitinib is (3S,4R)-3-ethyl-4-(3H-imidazo[1,2-a]pyrrolo[2,3-e]pyrazin-8-yl)-N-(2,2,2-trifluoro ethyl)pyrrolidine-1-carboxamide, and the structure thereof is shown in formula 1:

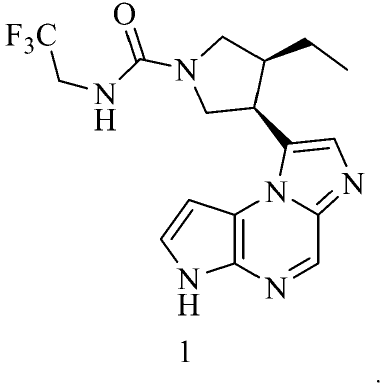

Several documents have disclosed preparation methods for Upadacitinib or a hydrate thereof, for example, CN108368121, CN102711476, CN115151529A, CN111217819, WO2023131978, and the like. However, there is still an urgent need in the art for a preparation method for Upadacitinib or a hydrate thereof that is easy to operate and capable of large-scale production.

### SUMMARY

In a first aspect, the present application provides a preparation method for Upadacitinib or a hydrate thereof, wherein the method comprises the following steps:
(1) reacting a compound of formula 2 with a compound of formula 3 in an organic solvent and water;
(2) mixing a reaction system obtained from step (1) with an inorganic base A and reacting to obtain a compound of formula 1 or a hydrate thereof,

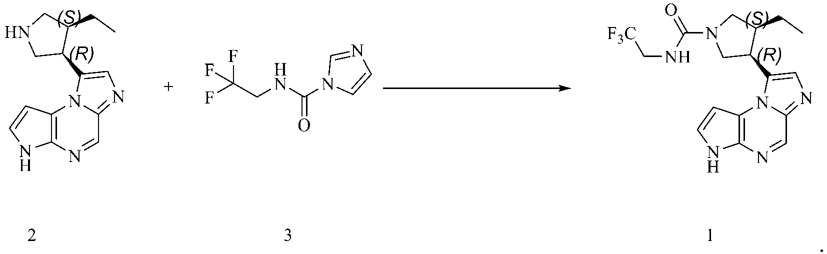

In a second aspect, the present application further provides a preparation method for Upadacitinib or a hydrate thereof, wherein the method comprises the following steps:
(1a) reacting an acid addition salt of a compound of formula 2 with an inorganic base B in an organic solvent and water to obtain the compound of formula 2;
(1) reacting the compound of formula 2 with a compound of formula 3 in an organic solvent and water;
(2) mixing a reaction system obtained from step (1) with an inorganic base A and reacting to obtain a compound of formula 1 or a hydrate thereof,

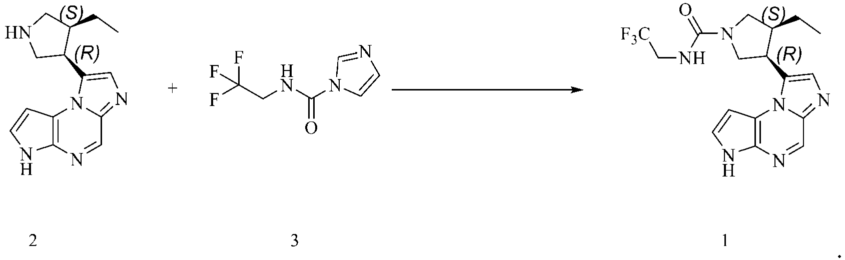

In a third aspect, the present application further provides a preparation method for a compound of formula 3, wherein N,N'-carbonyldiimidazole and trifluoroethylamine or a salt thereof are reacted in a solvent to obtain the compound of formula 3,

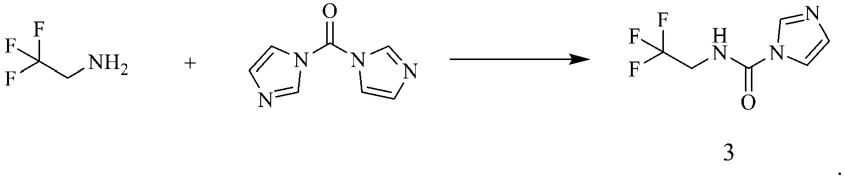

In a fourth aspect, the present application provides a preparation method for Upadacitinib or a hydrate thereof, wherein the method comprises the following steps:
(1b) reacting N,N'-carbonyldiimidazole with trifluoroethylamine or a salt thereof in a solvent to obtain a compound of formula 3;
(1a) reacting an acid addition salt of a compound of formula 2 with an inorganic base B in a solvent to obtain the compound of formula 2;
(1) reacting the compound of formula 2 with the compound of formula 3 in a solvent;
(2) mixing a reaction system obtained from step (1) with an inorganic base A and reacting to obtain a compound of formula 1-A, 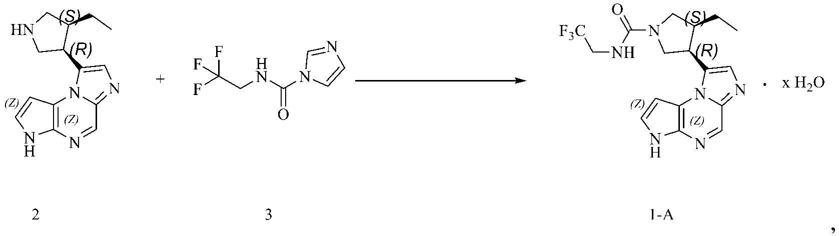 wherein x is 0 to 0.65.

In a fifth aspect, the present application further provides a preparation method for Upadacitinib hemihydrate, wherein: at 0 °C to 80 °C, adding an ethyl acetate solution of crude Upadacitinib to a mixed solvent of ethyl acetate and water, after the addition, maintaining at a temperature for 1 hour to 12 hours, cooling to -10 °C to 10 °C, and crystallizing for 0 hour to 4 hours.

In a sixth aspect, the present application further provides a high-purity Upadacitinib hemihydrate, wherein the content of a compound of formula 4 therein is less than 0.1%, and the purity of the Upadacitinib hemihydrate is higher than 98.0%,

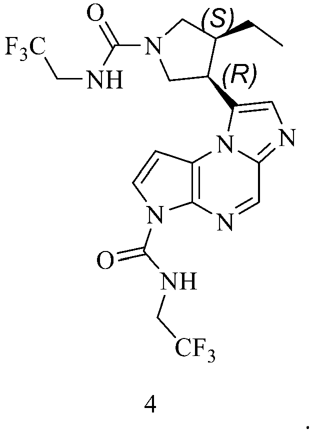

### DETAILED DESCRIPTION

In a first aspect, the present application provides a preparation method for Upadacitinib or a hydrate thereof, wherein the method comprises the following steps:
(1) reacting a compound of formula 2 with a compound of formula 3 in an organic solvent and water;
(2) mixing a reaction system obtained from step (1) with an inorganic base A and reacting to obtain a compound of formula 1 or a hydrate thereof,

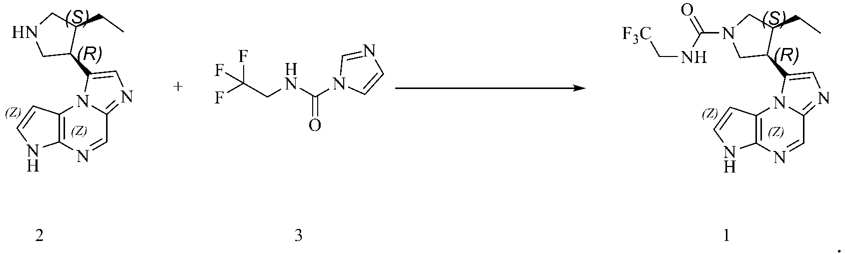

In some embodiments of the present application, the compound of formula 1 or the hydrate thereof is in a form of formula 1-A, 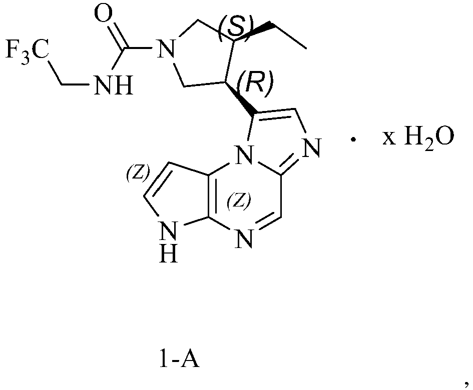 wherein x is 0 to 0.65.

In some typical embodiments of the present application, x is 0 to 0.5. In some more typical embodiments of the present application, x is 0 or 0.5. In some still more typical embodiments of the present application, x is 0; and in some most typical embodiments of the present application, x is 0.5.

In some embodiments of the present application, the molar ratio of the compound of formula 2 to the compound of formula 3 in step (1) is 1:1, 1:1.3, 1:1.4, 1:1.5, 1:1.6, 1:2, 1:2.1, 1:2.2, 1:2.5, 1:3, 1:4, or a range between any two of the above ratios. In some typical embodiments of the present application, the molar ratio of the compound of formula 2 to the compound of formula 3 in step (1) is 1:1 to 4. In some more typical embodiments of the present application, the molar ratio of the compound of formula 2 to the compound of formula 3 in step (1) is 1:1 to 3. In some still more typical embodiments of the present application, the molar ratio of the compound of formula 2 to the compound of formula 3 in step (1) is 1:1 to 2.5. In some most typical embodiments of the present application, the molar ratio of the compound of formula 2 to the compound of formula 3 in step (1) is 1:1.4 to 2.2. In some most typical embodiments of the present application, the molar ratio of the compound of formula 2 to the compound of formula 3 in step (1) is 1:1.4 to 1.5; and in some most typical embodiments of the present application, the molar ratio of the compound of formula 2 to the compound of formula 3 in step (1) is 1:2 to 2.2.

In some embodiments of the present application, the feeding mode of the compound of formula 3 in step (1) is dropwise addition, and the dropwise addition time is 0 hour to 10 hours. In some typical embodiments of the present application, the feeding mode of the compound of formula 3 in step (1) is dropwise addition, and the dropwise addition time is 1 hour to 5 hours. In some more typical embodiments of the present application, the feeding mode of the compound of formula 3 in step (1) is dropwise addition, and the dropwise addition time is 2 hours to 4 hours. In some still more typical embodiments of the present application, the feeding mode of the compound of formula 3 in step (1) is dropwise addition, and the dropwise addition time is 2 hours to 3 hours.

In some embodiments of the present application, the reaction temperature in step (1) is 0 °C to 50 °C. In some typical embodiments of the present application, the reaction temperature in step (1) is 10 °C to 40 °C. In some more typical embodiments of the present application, the reaction temperature in step (1) is 25 °C to 35 °C. In some still more typical embodiments of the present application, the reaction temperature in step (1) is 20 °C to 30 °C.

In some embodiments of the present application, the reaction time after the addition of the compound of formula 3 is completed in step (1) is 0 hour to 10 hours. In some typical embodiments of the present application, the reaction time after the addition of the compound of formula 3 is completed in step (1) is 0 hour to 5 hours. In some more typical embodiments of the present application, the reaction time after the addition of the compound of formula 3 is completed in step (1) is 1 hour to 3 hours. In some still more typical embodiments of the present application, the reaction time after the addition of the compound of formula 3 is completed in step (1) is 1 hour to 2 hours.

In some embodiments of the present application, the organic solvent in step (1) is one or more selected from the group consisting of tetrahydrofuran, 2-methyltetrahydrofuran, 1,4-dioxane, dichloromethane, and acetonitrile. In some typical embodiments of the present application, the organic solvent in step (1) is one or more selected from the group consisting of tetrahydrofuran, 2-methyltetrahydrofuran, and acetonitrile. In some more typical embodiments of the present application, the organic solvent in step (1) is one selected from the group consisting of tetrahydrofuran and 2-methyltetrahydrofuran. In some still more typical embodiments of the present application, the organic solvent in step (1) is tetrahydrofuran.

In some embodiments of the present application, the mass-to-volume ratio (g:mL) of the compound of formula 2 to the organic solvent in step (1) is 1:1, 1:8, 1:10, 1:11, 1:12, 1:16, 1:17, 1:18, 1:20, 1:22, 1:25, or a range between any two of the above ratios. In some typical embodiments, the mass-to-volume ratio (g:mL) of the compound of formula 2 to the organic solvent in step (1) is 1:1 to 25. In some more typical embodiments, the mass-to-volume ratio (g:mL) of the compound of formula 2 to the organic solvent in step (1) is 1:8 to 22. In some still more typical embodiments, the mass-to-volume ratio (g:mL) of the compound of formula 2 to the organic solvent in step (1) is 1:10 to 20. In some very typical embodiments, the mass-to-volume ratio (g:mL) of the compound of formula 2 to the organic solvent in step (1) is 1:10 to 18. In some most typical embodiments, the mass-to-volume ratio (g:mL) of the compound of formula 2 to the organic solvent in step (1) is 1:10 to 12. In some most typical embodiments, the mass-to-volume ratio (g:mL) of the compound of formula 2 to the organic solvent in step (1) is 1:11 to 18; and in some most typical embodiments, the mass-to-volume ratio (g:mL) of the compound of formula 2 to the organic solvent in step (1) is 1:16 to 18.

In some embodiments of the present application, the mass-to-volume ratio (g:mL) of the compound of formula 2 to water in step (1) is 1:1 to 16. In some typical embodiments of the present application, the mass-to-volume ratio (g:mL) of the compound of formula 2 to water in step (1) is 1:1 to 10. In some more typical embodiments of the present application, the mass-to-volume ratio (g:mL) of the compound of formula 2 to water in step (1) is 1:2 to 8.

In some embodiments of the present application, the inorganic base A in step (2) is selected from the group consisting of sodium hydroxide, potassium hydroxide, lithium hydroxide, sodium carbonate, potassium carbonate, and lithium carbonate. In some typical embodiments of the present application, the inorganic base A in step (2) is selected from the group consisting of sodium hydroxide, potassium hydroxide, and lithium hydroxide. In some more typical embodiments of the present application, the inorganic base A in step (2) is sodium hydroxide or potassium hydroxide. In some still more typical embodiments of the present application, the inorganic base A in step (2) is potassium hydroxide.

In some embodiments of the present application, the inorganic base A in step (2) is added in its solid form or in a form of an aqueous solution thereof. In some typical embodiments of the present application, the inorganic base A in step (2) is added in the form of an aqueous solution thereof. In some more typical embodiments of the present application, the inorganic base A in step (2) is added in the form of an aqueous solution thereof with a mass fraction of 5% to 50%. In some still more typical embodiments of the present application, the inorganic base A in step (2) is added in the form of an aqueous solution thereof with a mass fraction of 15% to 40%. In some very typical embodiments of the present application, the inorganic base A in step (2) is added in the form of an aqueous solution thereof with a mass fraction of 15% to 25%. In some most typical embodiments of the present application, the inorganic base A in step (2) is added in the form of an aqueous solution thereof with a mass fraction of 20%.

In some embodiments of the present application, in step (2), a molar ratio of the compound of formula 2 to the inorganic base A, calculated as monovalent basic anions of the inorganic base A, is 1:1 to 5. In some typical embodiments of the present application, in step (2), the molar ratio of the compound of formula 2 to the inorganic base A, calculated as monovalent basic anions of the inorganic base A, is 1:1 to 4. In some more typical embodiments of the present application, in step (2), the molar ratio of the compound of formula 2 to the inorganic base A, calculated as monovalent basic anions of the inorganic base A, is 1:1.5 to 2.5. In some still more typical embodiments of the present application, in step (2), the molar ratio of the compound of formula 2 to the inorganic base A, calculated as monovalent basic anions of the inorganic base A, is 1:2 to 2.5. In some very typical embodiments of the present application, in step (2), the molar ratio of the compound of formula 2 to the inorganic base A, calculated as monovalent basic anions of the inorganic base A, is 1:2 to 2.4. In some most typical embodiments of the present application, in step (2), the molar ratio of the compound of formula 2 to the inorganic base A, calculated as monovalent basic anions of the inorganic base A, is 1:2.1 to 2.4.

In some embodiments of the present application, the mixing temperature in step (2) is 0 °C to 50 °C. In some typical embodiments of the present application, the mixing temperature in step (2) is 10 °C to 50 °C. In some more typical embodiments of the present application, the mixing temperature in step (2) is 15 °C to 35 °C. In some still more typical embodiments of the present application, the mixing temperature in step (2) is 20 °C to 30 °C.

In some embodiments of the present application, the reaction time in step (2) is 0 hour to 10 hours. In some typical embodiments of the present application, the reaction time in step (2) is 1 hour to 6 hours. In some more typical embodiments of the present application, the reaction time in step (2) is 2 hours to 5 hours. In some still more typical embodiments of the present application, the reaction time in step (2) is 4 hours to 6 hours. In some still more typical embodiments of the present application, the reaction time in step (2) is 3 hours to 4 hours.

In some embodiments of the present application, after mixing and reacting in step (2), the method optionally further comprises adjusting the pH value to 3 to 6. In some typical embodiments of the present application, after mixing and reacting in step (2), the method further sequentially comprises adjusting the pH value to 4 to 6, extracting and performing layer separation. In some more typical embodiments of the present application, preferably, after mixing and reacting in step (2), the method further sequentially comprises adjusting the pH value to 4.8 to 6, extracting and performing layer separation. In some still more typical embodiments of the present application, after mixing and reacting in step (2), the method further sequentially comprises adjusting the pH value to 4.8 to 5.5, extracting with ethyl acetate and performing layer separation. In some still more typical embodiments of the present application, after mixing and reacting in step (2), the method further sequentially comprises adjusting the pH value to 5 to 6, extracting with ethyl acetate and performing layer separation.

In a second aspect, the preparation method for Upadacitinib or a hydrate thereof provided by the present application comprises the following steps:
(1a) reacting an acid addition salt of a compound of formula 2 with an inorganic base B in an organic solvent and water to obtain the compound of formula 2;
(1) reacting the compound of formula 2 with a compound of formula 3 in an organic solvent and water;
(2) mixing a reaction system obtained from step (1) with an inorganic base A and reacting to obtain a compound of formula 1 or a hydrate thereof,

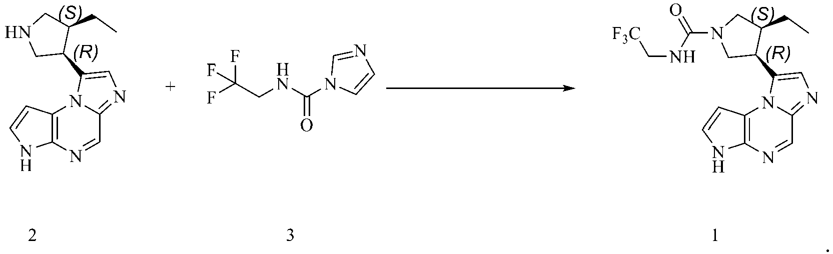

In some embodiments of the present application, step (1) and/or step (2) are as described above.

In some embodiments of the present application, the compound of formula 1 or the hydrate thereof is in the form of formula 1-A, 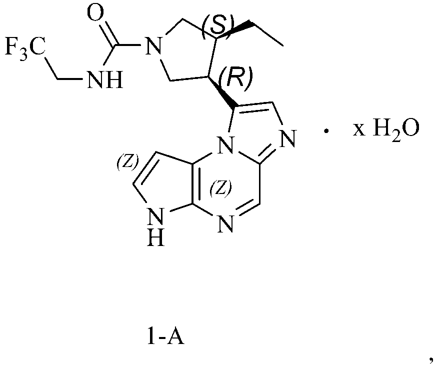 wherein x is 0 to 0.65.

In some typical embodiments of the present application, x is 0 to 0.5. In some more typical embodiments of the present application, x is 0 or 0.5. In some still more typical embodiments of the present application, x is 0; and in some most typical embodiments of the present application, x is 0.5.

In some embodiments of the present application, the acid addition salt of the compound of formula 2 in step (1a) is selected from the group consisting of a hydrobromide, a hydrochloride, a sulfate, and a phosphate of the compound of formula 2. In some typical embodiments of the present application, the acid addition salt of the compound of formula 2 in step (1a) is selected from the group consisting of the hydrobromide, the hydrochloride, and the sulfate of the compound of formula 2. In some more typical embodiments of the present application, the acid addition salt of the compound of formula 2 in step (1a) is the hydrobromide or the hydrochloride of the compound of formula 2. In some still more typical embodiments of the present application, the acid addition salt of the compound of formula 2 in step (1a) is the hydrobromide of the compound of formula 2.

In some embodiments of the present application, the inorganic base B in step (1a) is selected from the group consisting of sodium hydroxide, potassium hydroxide, lithium hydroxide, sodium carbonate, potassium carbonate, and lithium carbonate. In some typical embodiments of the present application, the inorganic base B in step (1a) is selected from the group consisting of sodium hydroxide, potassium hydroxide, and lithium hydroxide. In some more typical embodiments of the present application, the inorganic base B in step (1a) is sodium hydroxide or potassium hydroxide. In some still more typical embodiments of the present application, the inorganic base B in step (1a) is potassium hydroxide.

In some embodiments of the present application, in step (1a), the molar ratio of the acid addition salt of the compound of formula 2 to the inorganic base B, calculated as monovalent basic anions of inorganic base B, is 1:1 to 5. In some typical embodiments of the present application, in step (1a), the molar ratio of the acid addition salt of the compound of formula 2 to the inorganic base B, calculated as monovalent basic anions of the inorganic base B, is 1:1 to 4. In some more typical embodiments of the present application, in step (1a), the molar ratio of the acid addition salt of the compound of formula 2 to the inorganic base B, calculated as monovalent basic anions of the inorganic base B, is 1:1.5 to 2.5. In some still more typical embodiments of the present application, in step (1a), the molar ratio of the acid addition salt of the compound of formula 2 to the inorganic base B, calculated as monovalent basic anions of the inorganic base B, is 1:1.8 to 2.2. In some most typical embodiments of the present application, in step (1a), the molar ratio of the acid addition salt of the compound of formula 2 to the inorganic base B, calculated as monovalent basic anions of the inorganic base B, is 1:2. In some most typical embodiments of the present application, in step (1a), the inorganic base B is potassium hydroxide, and the molar ratio of the acid addition salt of the compound of formula 2 to the inorganic base B is 1:2.

In some embodiments of the present application, the pH of the reaction system in step (1a) is adjusted to 8 to 9 by adding the inorganic base B.

In some embodiments of the present application, in step (1a), the inorganic base B is added in a form of an aqueous solution thereof. In some typical embodiments, the inorganic base B is added in the form of an aqueous solution thereof, and the mass of the inorganic base B accounts for 20% of the mass of the aqueous solution thereof. In some more typical embodiments, the inorganic base B is added in the form of an aqueous solution of potassium hydroxide thereof, and the mass of potassium hydroxide accounts for 20% of the mass of the aqueous solution thereof.

In some embodiments of the present application, the organic solvent in step (1a) is one or more selected from the group consisting of tetrahydrofuran, 2-methyltetrahydrofuran, 1,4-dioxane, dichloromethane, and acetonitrile. In some typical embodiments of the present application, the organic solvent in step (1a) is one or more selected from the group consisting of tetrahydrofuran, 2-methyltetrahydrofuran, and acetonitrile. In some more typical embodiments of the present application, the organic solvent in step (1a) is tetrahydrofuran or 2-methyltetrahydrofuran. In some still more typical embodiments of the present application, the organic solvent in step (1a) is tetrahydrofuran.

In some embodiments of the present application, the mass-to-volume ratio (g:mL) of the acid addition salt of the compound of formula 2, calculated as its free base, to the organic solvent in step (1a) is 1:1 to 16. In some typical embodiments, the mass-to-volume ratio (g:mL) of the acid addition salt of the compound of formula 2, calculated as its free base, to the organic solvent in step (1a) is 1:1.5 to 5. In some more typical embodiments, the mass-to-volume ratio (g:mL) of the acid addition salt of the compound of formula 2, calculated as its free base, to the organic solvent in step (1a) is 1:2.3 to 5. In some still more typical embodiments, the mass-to-volume ratio (g:mL) of the acid addition salt of the compound of formula 2, calculated as its free base, to the organic solvent in step (1a) is 1:2.3 to 4. In some most typical embodiments, the mass-to-volume ratio (g:mL) of the acid addition salt of the compound of formula 2, calculated as its free base, to the organic solvent in step (1a) is 1:2.3 to 2.6.

In some embodiments of the present application, the reaction temperature in step (1a) is 0 °C to 50 °C. In some typical embodiments of the present application, the reaction temperature in step (1a) is 10 °C to 50 °C. In some more typical embodiments of the present application, the reaction temperature in step (1a) is 15 °C to 35 °C. In some still more typical embodiments of the present application, the reaction temperature in step (1a) is 20 °C to 30 °C.

In a third aspect, the present application further provides a preparation method for a compound of formula 3, wherein N,N'-carbonyldiimidazole and trifluoroethylamine or a salt thereof are reacted in a solvent to obtain the compound of formula 3,

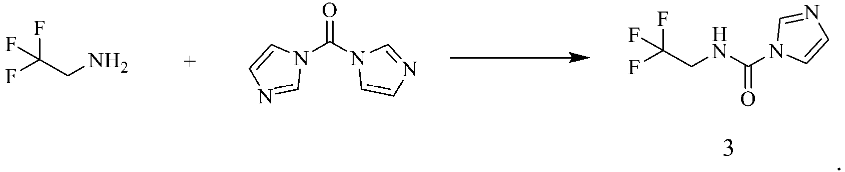

In some embodiments of the present application, the reaction temperature of the reaction is -10 °C to 30 °C. In some typical embodiments of the present application, the reaction temperature of the reaction is -10 °C to 10 °C. In some more typical embodiments of the present application, the reaction temperature of the reaction is -5 °C to 5 °C.

In some embodiments of the present application, the trifluoroethylamine or the salt thereof is trifluoroethylamine or the hydrochloride thereof. In some typical embodiments of the present application, the trifluoroethylamine or the salt thereof is trifluoroethylamine or the monohydrochloride thereof. In some more typical embodiments of the present application, the trifluoroethylamine or the salt thereof is trifluoroethylamine free base. In some still more typical embodiments of the present application, the trifluoroethylamine or the salt thereof is trifluoroethylamine monohydrochloride.

In some embodiments of the present application, the trifluoroethylamine or the salt thereof is an acid addition salt of trifluoroethylamine, and the above preparation method further comprises adding a base to the reaction solution after the reaction. In some typical embodiments of the present application, the trifluoroethylamine or the salt thereof is an acid addition salt of trifluoroethylamine, and the above preparation method further comprises adding an aqueous solution of a base to the reaction solution after the reaction. In some more typical embodiments of the present application, the trifluoroethylamine or the salt thereof is an acid addition salt of trifluoroethylamine, and the above preparation method further comprises adding an aqueous solution of potassium hydroxide to the reaction solution after the reaction. In some still more typical embodiments of the present application, the trifluoroethylamine or the salt thereof is an acid addition salt of trifluoroethylamine, and the above preparation method further comprises adding a 20% aqueous solution of potassium hydroxide to the reaction solution after the reaction. In some most typical embodiments of the present application, the trifluoroethylamine or the salt thereof is trifluoroethylamine monohydrochloride, and the method further comprises adding a 20% aqueous solution of potassium hydroxide to the reaction solution after the reaction, and the molar ratio of trifluoroethylamine monohydrochloride to potassium hydroxide is 1:1.

In some embodiments of the present application, the molar ratio of the trifluoroethylamine or the salt thereof to N,N'-carbonyldiimidazole is 1:0.5, 1:0.8, 1:0.84, 1:0.9, 1:0.94, 1:0.95, 1:1, 1:1.5, 1:2, or a range between any two of the above ratios. In some typical embodiments of the present application, the molar ratio of the trifluoroethylamine or the salt thereof to N,N'-carbonyldiimidazole is 1:0.5 to 2. In some more typical embodiments of the present application, the molar ratio of the trifluoroethylamine or the salt thereof to N,N'-carbonyldiimidazole is 1:0.5 to 1.5. In some still more typical embodiments of the present application, the molar ratio of the trifluoroethylamine or the salt thereof to N,N'-carbonyldiimidazole is 1:0.5 to 1. In some very typical embodiments of the present application, the molar ratio of the trifluoroethylamine or the salt thereof to N,N'-carbonyldiimidazole is 1:0.8 to 1. In some most typical embodiments of the present application, the molar ratio of the trifluoroethylamine or the salt thereof to N,N'-carbonyldiimidazole is 1:0.85 to 0.95. In some most typical embodiments of the present application, the molar ratio of the trifluoroethylamine or the salt thereof to N,N'-carbonyldiimidazole is 1:0.9 to 0.95.

In some embodiments of the present application, the reaction solvent is one or more selected from the group consisting of tetrahydrofuran, 2-methyltetrahydrofuran, 1,4-dioxane, dichloromethane, and acetonitrile. In some typical embodiments of the present application, the reaction solvent is one or more selected from the group consisting of tetrahydrofuran, 2-methyltetrahydrofuran, and acetonitrile. In some more typical embodiments of the present application, the reaction solvent is tetrahydrofuran or 2-methyltetrahydrofuran. In some still more typical embodiments of the present application, the reaction solvent is tetrahydrofuran.

In some embodiments of the present application, the mass-to-volume ratio (g:mL) of N,N'-carbonyldiimidazole to the solvent is 1:1, 1:5, 1:7, 1:8, 1:9, 1:10, 1:11, 1:12, 1:14, 1:20, 1:25, or a range between any two of the above ratios. In some typical embodiments of the present application, the mass-to-volume ratio (g:mL) of N,N'-carbonyldiimidazole to the solvent is 1:1 to 25. In some more typical embodiments of the present application, the mass-to-volume ratio (g:mL) of N,N'-carbonyldiimidazole to the solvent is 1:5 to 20. In some still more typical embodiments of the present application, the mass-to-volume ratio (g:mL) of N,N'-carbonyldiimidazole to the solvent is 1:7 to 14. In some very typical embodiments of the present application, the mass-to-volume ratio (g:mL) of N,N'-carbonyldiimidazole to the solvent is 1:8 to 12. In some most typical embodiments of the present application, the mass-to-volume ratio (g:mL) of N,N'-carbonyldiimidazole to the solvent is 1:7 to 10. In some most typical embodiments of the present application, the mass-to-volume ratio (g:mL) of N,N'-carbonyldiimidazole to the solvent is 1:8 to 10. In some most typical embodiments of the present application, the mass-to-volume ratio (g:mL) of N,N'-carbonyldiimidazole to the solvent is 1:9 to 10.

In some embodiments of the present application, the reaction time of the reaction is 0.5 hour to 3 hours. In some typical embodiments of the present application, the reaction time of the reaction is 1 hour to 2 hours.

In some embodiments of the present application, the reaction is carried out under a nitrogen atmosphere.

In a fourth aspect, the present application further provides a preparation method for Upadacitinib or a hydrate thereof, wherein the method comprises the following steps:
(1b) reacting N,N'-carbonyldiimidazole with trifluoroethylamine or a salt thereof in a solvent to obtain a compound of formula 3;
(1a) reacting an acid addition salt of a compound of formula 2 with an inorganic base B in a solvent to obtain the compound of formula 2;
(1) reacting the compound of formula 2 with the compound of formula 3 in an organic solvent and water;
(2) mixing a reaction system obtained from step (1) with an inorganic base A and reacting to obtain a compound of formula 1-A, 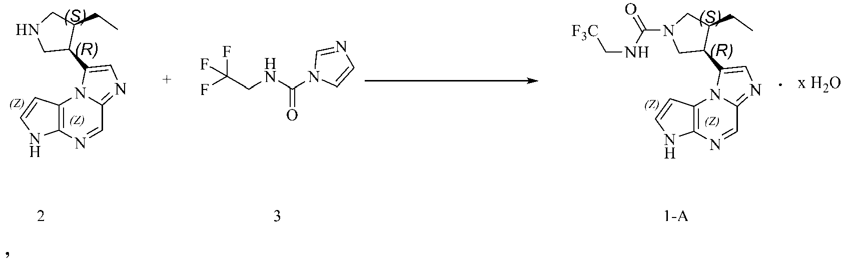 wherein x is 0 to 0.65.

In some embodiments of the present application, step (1b), step (1a), step (1) and/or step (2) are as described above.

In some typical embodiments of the present application, x is 0 to 0.5. In some more typical embodiments of the present application, x is 0 or 0.5. In some still more typical embodiments of the present application, x is 0; and in some most typical embodiments of the present application, x is 0.5.

In some embodiments of the present application, the molar ratio of the acid addition salt of the compound of formula 2 in step (1a) to the trifluoroethylamine or the salt thereof in step (1b) is 1:1, 1:1.5, 1:1.6, 1:1.8, 1:2, 1:2.3, 1:2.4, 1:2.5, 1:3, or a range between any two of the above ratios. In some typical embodiments of the present application, the molar ratio of the acid addition salt of the compound of formula 2 in step (1a) to the trifluoroethylamine or the salt thereof in step (1b) is 1:1 to 3. In some more typical embodiments of the present application, the molar ratio of the acid addition salt of the compound of formula 2 in step (1a) to the trifluoroethylamine or the salt thereof in step (1b) is 1:1 to 2.5. In some still more typical embodiments of the present application, the molar ratio of the acid addition salt of the compound of formula 2 in step (1a) to the trifluoroethylamine or the salt thereof in step (1b) is 1:1 to 2. In some very typical embodiments of the present application, the molar ratio of the acid addition salt of the compound of formula 2 in step (1a) to the trifluoroethylamine or the salt thereof in step (1b) is 1:1.5 to 2.5. In some most typical embodiments of the present application, the molar ratio of the acid addition salt of the compound of formula 2 in step (1a) to the trifluoroethylamine or the salt thereof in step (1b) is 1:1.5 to 1.8. In some most typical embodiments of the present application, the molar ratio of the acid addition salt of the compound of formula 2 in step (1a) to the trifluoroethylamine or the salt thereof in step (1b) is 1:2.3 to 2.5.

In some embodiments of the present application, the resultant of step (1b) is used directly in the next step without purification.

In a fifth aspect, the present application further provides a preparation method for Upadacitinib hemihydrate, wherein: at 0 °C to 80 °C, adding an ethyl acetate solution of crude Upadacitinib to a mixed solvent of ethyl acetate and water, after the addition, maintaining at a temperature for 1 hour to 12 hours, cooling to -10 °C to 10 °C, and crystallizing for 0 hour to 4 hours.

In some embodiments of the present application, the crude Upadacitinib is Upadacitinib free base or a Upadacitinib hydrate. In some typical embodiments of the present application, the crude Upadacitinib is a Upadacitinib free base. In some typical embodiments of the present application, the crude Upadacitinib is a Upadacitinib hydrate. In some more typical embodiments of the present application, the crude Upadacitinib is a Upadacitinib hemihydrate.

In some embodiments of the present application, the mass ratio (kg:kg) of the crude Upadacitinib to ethyl acetate in the ethyl acetate solution of crude Upadacitinib is 1:1, 1:2, 1:2.2, 1:2.5, 1:3, 1:3.2, 1:6, 1:9, 1:10, or a range between any two of the above ratios. In some typical embodiments of the present application, the mass ratio (kg:kg) of the crude Upadacitinib to ethyl acetate in the ethyl acetate solution of crude Upadacitinib is 1:1 to 10. In some more typical embodiments of the present application, the mass ratio (kg:kg) of the crude Upadacitinib to ethyl acetate in the ethyl acetate solution of crude Upadacitinib is 1:2 to 9. In some still more typical embodiments of the present application, the mass ratio (kg:kg) of the crude Upadacitinib to ethyl acetate in the ethyl acetate solution of crude Upadacitinib is 1:2 to 6. In some very typical embodiments of the present application, the mass ratio (kg:kg) of the crude Upadacitinib to ethyl acetate in the ethyl acetate solution of crude Upadacitinib is 1:2.2 to 3.2. In some most typical embodiments of the present application, the mass ratio (kg:kg) of the crude Upadacitinib to ethyl acetate in the ethyl acetate solution of crude Upadacitinib is 1:2.5 to 3.

In some embodiments of the present application, the molar ratio of the crude Upadacitinib to water is 1:0.5 to 4. In some typical embodiments of the present application, the molar ratio of the crude Upadacitinib to water is 1:0.5 to 2. In some more typical embodiments of the present application, the molar ratio of the crude Upadacitinib to water is 1:0.8 to 2. In some still more typical embodiments of the present application, the molar ratio of the crude Upadacitinib to water is 1:0.8 to 1.2.

In some embodiments of the present application, the total mass of ethyl acetate refers to the total amount of ethyl acetate used in the ethyl acetate solution of crude Upadacitinib and the mixed solvent of ethyl acetate and water in the above preparation method, and the mass ratio of water to the total mass of ethyl acetate is 1:200 to 1:32. In some more typical embodiments of the present application, the total mass of ethyl acetate refers to the total amount of ethyl acetate used in the ethyl acetate solution of crude Upadacitinib and the mixed solvent of ethyl acetate and water in the above preparation method, and the mass ratio of water to the total mass of ethyl acetate is 1:200 to 1:50. In some still more typical embodiments of the present application, the total mass of ethyl acetate refers to the total amount of ethyl acetate used in the ethyl acetate solution of crude Upadacitinib and the mixed solvent of ethyl acetate and water in the above preparation method, and the mass ratio of water to the total mass of ethyl acetate is 1:100 to 1:50. In some most typical embodiments of the present application, the total mass of ethyl acetate refers to the total amount of ethyl acetate used in the ethyl acetate solution of crude Upadacitinib and the mixed solvent of ethyl acetate and water in the above preparation method, and the mass ratio of water to the total mass of ethyl acetate is 1:100 to 1:78.

In some embodiments of the present application, the addition mode of the ethyl acetate solution of crude Upadacitinib is dropwise addition.

In some embodiments of the present application, the method further comprises that the dropwise addition time of the ethyl acetate solution of crude Upadacitinib is 1 hour to 6 hours. In some typical embodiments of the present application, the method further comprises that the dropwise addition time of the ethyl acetate solution of crude Upadacitinib is 1 hour to 5 hours. In some more typical embodiments of the present application, the method further comprises that the dropwise addition time of the ethyl acetate solution of crude Upadacitinib is 2 hours to 5 hours. In some still more typical embodiments of the present application, the method further comprises that the dropwise addition time of the ethyl acetate solution of crude Upadacitinib is 3 hours to 4 hours.

In some embodiments of the present application, the maintained temperature is 0 °C to 50 °C. In some typical embodiments of the present application, the maintained temperature is 20 °C to 50 °C. In some more typical embodiments of the present application, the maintained temperature is 30 °C to 45 °C. In some still more typical embodiments of the present application, the maintained temperature is 40 °C to 45 °C.

In some embodiments of the present application, the period of time at the maintained temperature is 1 hour to 10 hours. In some typical embodiments of the present application, the period of time at the maintained temperature is 2 hours to 8 hours. In some more typical embodiments of the present application, the period of time at the maintained temperature is 3 hours to 6 hours. In some still more typical embodiments of the present application, the period of time at the maintained temperature is 4 hours to 5 hours.

In some embodiments of the present application, the temperature after cooling is -5 °C to 10 °C. In some typical embodiments of the present application, the temperature after cooling is -5 °C to 5 °C. In some more typical embodiments of the present application, the temperature after cooling is 0 °C to 5 °C.

In some embodiments of the present application, the cooling rate is 0.15 °C/min to 1.0 °C/min. In some typical embodiments of the present application, the cooling rate is 0.15 °C/min to 0.75 °C/min. In some more typical embodiments of the present application, the cooling rate is 0.15 °C/min to 0.5 °C/min. In some still more typical embodiments of the present application, the cooling rate is 0.15 °C/min to 0.3 °C/min.

In some embodiments of the present application, the crystallization time is 0 hour to 3 hours. In some typical embodiments of the present application, the crystallization time is 0.5 hour to 3 hours. In some still more typical embodiments of the present application, the crystallization time is 0.5 hour to 2 hours.

In some embodiments of the present application, the preparation method optionally further comprises decolorizing with activated carbon before the crude Upadacitinib is dissolved in ethyl acetate. In some typical embodiments of the present application, the preparation method optionally further comprises decolorizing with activated carbon in a mixed solvent of ethyl acetate and ethanol or in a single solvent of ethyl acetate before the crude Upadacitinib is dissolved in ethyl acetate. In some still more typical embodiments of the present application, the preparation method optionally further comprises decolorizing with activated carbon in a mixed solvent of ethyl acetate and ethanol or in a single solvent of ethanol, filtering, and distilling the filtrate to dryness before the crude Upadacitinib is dissolved in ethyl acetate.

In the present application, the source of the crude Upadacitinib includes, but is not limited to, being prepared according to prior art such as CN108368121, CN102711476, CN115151529A, CN111217819 or WO2023131978, or being prepared by the preparation method for Upadacitinib of the present application.

In a sixth aspect, the present application further provides a high-purity Upadacitinib hemihydrate, wherein the content of a compound of formula 4 therein is less than 0.1%, and the purity of the Upadacitinib hemihydrate is higher than 98.0%,

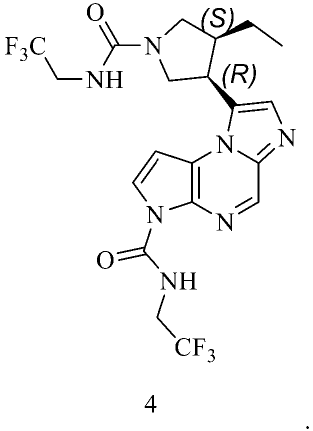

In some embodiments of the present application, the content of the compound of formula 4 is less than 0.05%. In some typical embodiments of the present application, the content of the compound of formula 4 is less than 0.03%. In some more typical embodiments of the present application, the content of the compound of formula 4 is less than 0.015%.

In some embodiments of the present application, the purity of the Upadacitinib hemihydrate is higher than 98.5%. In some typical embodiments of the present application, the purity of the Upadacitinib hemihydrate is higher than 99.0%. In some more typical embodiments of the present application, the purity of the Upadacitinib hemihydrate is higher than 99.5%. In some still more typical embodiments of the present application, the purity of the Upadacitinib hemihydrate is higher than 99.8%.

In the present application, the purity of Upadacitinib and the hydrate thereof and the content of the compound of formula 4 can both be determined by the following method:

### 1. Chromatographic Conditions

**Table 1 Chromatographic Conditions**

| Instrument | High performance liquid chromatograph equipped with an ultraviolet detector | | |
|---|---|---|---|
| Chromatogr aphic column | ACE-EXCEL C18-PFP 4.6*150 mm 3.0 µm | | |
| | Cat No.: EXL-1110-1546U | | |
| Buffer | 1.1 g of ammonium acetate was dissolved in 1000 mL of water, mixed well, sonicated, and filtered. | | |
| Mobile phase A | Buffer : Mobile phase B = 95:5 (%V/V) | | |
| Mobile phase B | Acetonitrile : Isopropanol = 85:15 (%V/V) | | |
| Detection wavelength | 233 nm | | |
| Flow rate | 1.0 mL/min | | |
| Injection volume | 10 µL | | |
| Column temperature | 40 °C | | |
| Peak width | >0.1 min (2 s response time) (2.5 Hz) | | |

| Acquisition time: | 50 min | | |
|---|---|---|---|
| Gradient | Time (min) | Mobile phase A (% V/V) | Mobile phase B (%V/V) |
| | 0.00 | 100 | 0 |
| | 10 | 79 | 21 |
| | 30 | 58 | 42 |
| | 40 | 31 | 69 |
| | 40.1 | 100 | 0 |
| | 50 | 100 | 0 |
| Chromatogr aphic column washing program | Time (min) | Water (% V/V) | Acetonitrile (%V/V) |
| | 0.00 | 95 | 5 |
| | 60 | 95 | 5 |
| | 120 | 5 | 95 |
| | 180 | 5 | 95 |

### 2. Reagents

Ammonium acetate: chromatographic grade or analytical grade
Acetonitrile: chromatographic grade
Isopropanol: chromatographic grade
Water: purified water

### 3. Solution Preparation

Diluent: Acetonitrile : Water = 50:50 (%V/V)
Blank solution: Diluent
Solution of test sample: 25 mg of the test sample was weighed, accurately weighed into a 50 mL volumetric flask, dissolved and diluted to volume with the diluent, and mixed well.

### 4. Procedure

The blank solution was injected for 1 injection, and the solution of test sample was injected for 1 injection, respectively, and the chromatographic process was recorded.

### 5. Calculation

Calculated by the area normalization method, the detection limit of the aforementioned HPLC method is 0.015%.

The preparation method provided by the present application can obtain the high-purity Upadacitinib hemihydrate in high yield, which does not require strict control of the change in the pH value of the reaction system during the reaction process, and has the advantages of easy operation and readily available product. More importantly, the method is not only suitable for small-scale preparation of Upadacitinib hemihydrate, but also can stably achieve scale-up production and large-scale production. The Upadacitinib hemihydrate prepared by the present application has good purity.

Unless otherwise specified, the terms of the present application have the following meanings:
The solution content percentages are all mass fractions, for example, "20% KOH aqueous solution (11.14 g)" means that the mass of the solute KOH accounts for 20% of the total mass of the solute KOH and the solvent water, i.e., the mass of the solute KOH being 2.228 g.

"CDI" is N,N'-carbonyldiimidazole.

"EA" is ethyl acetate.

"KOH" is potassium hydroxide.

"THF" is tetrahydrofuran.

"Crude product" refers to a substance that may contain impurities and is to be further purified. In the present application, "crude Upadacitinib" refers to the substance obtained after preliminary purification in the process of synthesizing Upadacitinib hemihydrate.

"Higher than" means "≥".

"Less than" means "≤".

In the present application, silica gel thin-layer chromatography can be used to confirm the reaction endpoint.

In the present application, the feed amount of a mixture represents the total mass or total volume of all substances constituting the mixture, for example, "20% KOH aqueous solution (11.14 g)" means that the total mass of the solute KOH and the solvent water is 11.14 g, i.e., the total mass of the KOH aqueous solution being 11.14 g.

In the present application, "the preparation method is the same as that of Example n" and "prepared with reference to Example n" mean that only the reactants, catalysts and/or solvents required for the reaction are scaled up or down in the same molar ratio as in Example n, and the remaining conditions are substantially unchanged, so as to obtain the required amount of the compound or mixture. The "remaining conditions" include, but are not limited to, the reaction environment, reaction temperature, reaction time, feeding mode or post-treatment operation, and the like. "Example n" refers to the example number n used as the reference basis.

In the present application, when the preparation method includes other preceding steps before step (1), the amount of water used in step (1) includes the amount of water in the aqueous solution of the base added in step (1a) and/or step (1b) of the preparation method; and the amount of the organic solvent used in step (1) includes the amount of the organic solvent added in step (1a) and/or step (1b) of the preparation method.

In the present application, those skilled in the art can readily understand that the materials can be added in various ways without affecting the essence of the application. For example, the addition mode of the N,N'-carbonyldiimidazole includes, but is not limited to, direct addition, or addition in the form of a solution thereof; and the addition mode of the trifluoroethylamine or the salt thereof includes, but is not limited to, direct addition, or addition in the form of a solution thereof.

Those skilled in the art can readily understand that experimental errors, technical errors and instrumental errors may cause numerical fluctuations in the technical solutions, but they still substantially fall within the content of the present application.

Those skilled in the art can readily understand that the operational chronological order of step (1b) and step (1a) in the present application can be adjusted according to actual needs, for example, they can be performed simultaneously or in any swapped order.

### EXAMPLES

The present application will be further illustrated by the following specific examples, but they are not intended to limit the protection scope of the present application. Without departing from the concept of the present application, any modifications or changes to the present application that can be readily implemented by those skilled in the art will fall within the scope of the present application.

### Example 1-1. Synthesis of N-(2,2,2-trifluoroethyl)-1H-imidazole-1-carboxamide (Formula 3)

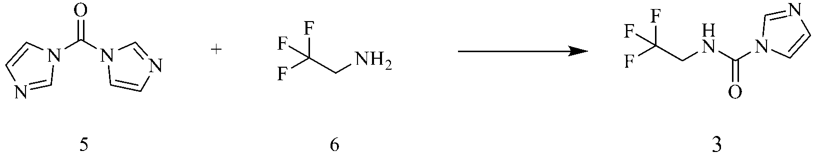

CDI (4.84 g) and THF (20 mL) were added to a reaction flask, under nitrogen protection, the temperature was cooled to -5 °C to 5 °C. Then trifluoroethylamine free base (3.17 g) and THF (26 mL) were added. The system temperature was controlled to ≤5 °C during the addition process, and the temperature was maintained at -5 °C to 5 °C for 1 h to 2 h to obtain a solution of the compound of formula 3. The solution was used directly in the next step without purification.

### Example 1-2. Synthesis of Upadacitinib hemihydrate

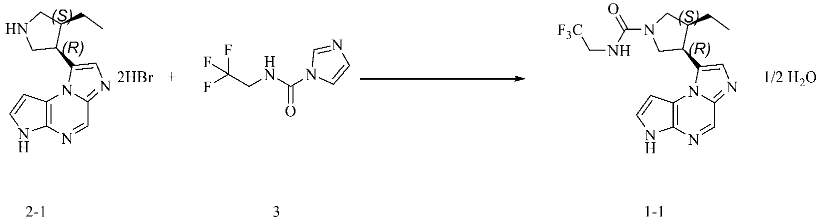

The compound of formula 2-1 (8.31 g), THF (13 mL), water (13 mL) and 20% KOH aqueous solution (11.14 g) were added to a reaction flask, and stirred to dissolve. The reaction temperature was controlled at 20 °C to 30 °C. The solution of the compound of formula 3 obtained in Example 1-1 was added dropwise, and the dropwise addition time was 2 h to 3 h. After completion of the dropwise addition, the reaction was carried out at 20 °C to 30 °C for 1 h to 2 h. After completion of the reaction, 20% KOH aqueous solution (13.0 g) was added, and the reaction was carried out at 20 °C to 30 °C for 3 h to 4 h. After completion of the reaction, 36% hydrochloric acid aqueous solution was added to adjust the pH to 4.8 to 5.5. Then, EA (26 mL) was added for extraction. The aqueous layer was separated and removed. The organic layer was washed successively with saturated NaHCO₃ aqueous solution (26 mL) and 20% NaCl aqueous solution (13 mL), and concentrated. No compound of formula 4 was detected. EA (37.4 g) and water (0.74 g) were added for crystallization to obtain 6.68 g of Upadacitinib hemihydrate with a purity of 99.74%, ESI-MS m/z: 390 [M+H]⁺, and no compound of formula 4 was detected.

### Example 2-1. Scale-up synthesis of N-(2,2,2-trifluoroethyl)-1H-imidazole-1-carboxamide (Formula 3)

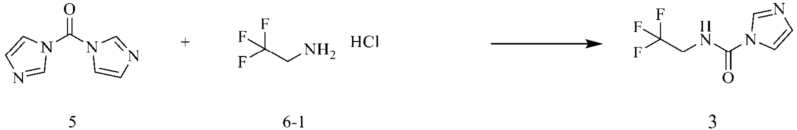

CDI (13.5 kg) and THF (92.8 kg) were added to a reaction flask. Under nitrogen protection, the temperature was cooled to -5 °C to 5 °C, and trifluoroethylamine hydrochloride (12.1 kg) was added in 10 batches. The system temperature was controlled at -5 °C to 5 °C during the addition process. The reaction was maintained at the temperature for 1 h to 2 h. After completion of the maintaining, 20% KOH aqueous solution (25 kg) was added, and the system temperature was controlled at -5 °C to 5 °C during the addition process. After completion of the dropwise addition, the temperature was maintained for 0.5 to 1 h. The mixture was filtered. The filter cake was rinsed with THF (20.2 kg), and the filtrates were combined to obtain a solution of the compound of formula 3.

### Example 2-2. Scale-up synthesis of Upadacitinib hemihydrate

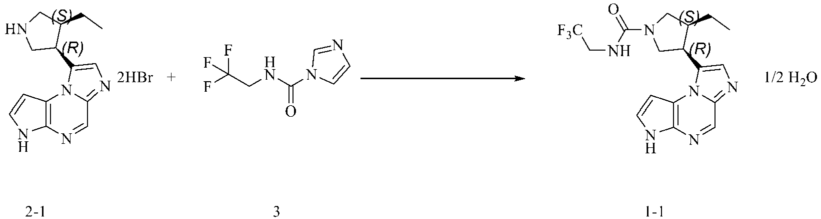

The compound of formula 2-1 (23.2 kg), THF (32.3 kg), water (36.3 kg) and 20% KOH aqueous solution (31.1 kg) were added to a reaction flask, and stirred to dissolve. The reaction temperature was controlled at 20 °C to 30 °C. The solution of the compound of formula 3 obtained in Example 2-1 was added dropwise, and the dropwise addition time was 2 h to 3 h. After completion of the dropwise addition, the reaction was carried out at 20 °C to 30 °C for 1 h to 2 h. After completion of the reaction, 20% KOH aqueous solution (36.18 kg) was added, and the reaction was carried out at 20 °C to 30 °C for 3 h to 4 h. After completion of the reaction, 36% hydrochloric acid aqueous solution was added to adjust the pH to 4.8 to 5.5. Then, EA (66.8 kg) was added for extraction. The aqueous layer was separated and removed. The organic layer was washed successively with saturated NaHCO₃ aqueous solution (73 kg) and 20% NaCl aqueous solution (42.2 kg), and concentrated. No compound of formula 4 was detected. EA (104.4 kg) and water (2.06 kg) were added, and the temperature was maintained at 40 °C to 45 °C. Then, the temperature was cooled to 0 to 5 °C, crystallization was carried out for 0.5 h to 1 h. Solid-liquid separation was performed, and the filter cake was dried to obtain 19.1 kg of Upadacitinib hemihydrate with a purity of 99.90%, ESI-MS m/z: 390 [M+H]⁺, and no compound of formula 4 was detected.

### Example 3-1. Synthesis of N-(2,2,2-trifluoroethyl)-1H-imidazole-1-carboxamide (Formula 3)

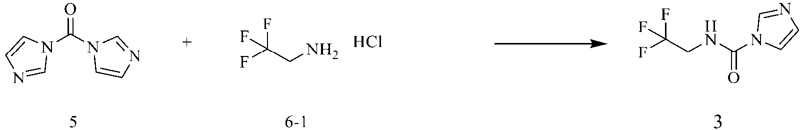

CDI (18.19 kg) and THF (136.44 kg) were added to a reaction flask and stirred. The temperature was controlled at 0 to 5 °C. Trifluoroethylamine hydrochloride (17.74 kg) was added in batches, and the temperature was maintained for 1 h to 2 h. After completion of the reaction, 20% KOH aqueous solution (36.61 kg) was slowly added dropwise. After completion of the dropwise addition, the mixture was filtered, and the filter cake was rinsed with THF (29.79 kg). The filtrates were combined to obtain a solution of the compound of formula 3.

### Example 3-2. Synthesis of Upadacitinib hemihydrate

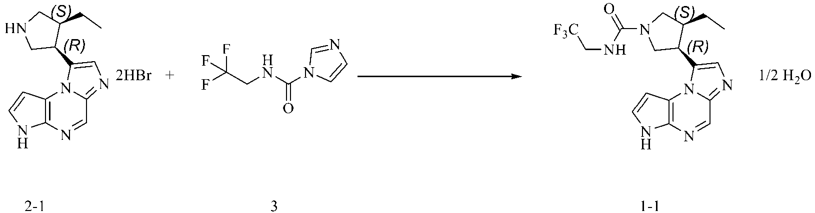

The compound of formula 2-1 (22.74 kg), THF (47.75 kg), and water (36.61 kg) were added to a reaction flask. The internal temperature was controlled at 15 to 25 °C. The pH was adjusted to 8 to 9 with 20% KOH aqueous solution. The temperature was controlled at 20 to 25 °C, and the solution of the compound of formula 3 obtained in Example 3-1 was slowly added dropwise. After completion of the dropwise addition, the pH was adjusted to 8 to 9 with 20% KOH aqueous solution, and the reaction was maintained at 20 to 25 °C for 1 to 3 hours. After completion of the reaction, the temperature was controlled at 20 to 30 °C. 20% KOH aqueous solution (32.06 kg) was added, and the reaction was maintained for 4 to 6 hours. After completion of the reaction, the temperature was cooled to 5 to 15 °C. The pH was adjusted to 5 to 6 with hydrochloric acid. Ethyl acetate (65.49 kg) was added. The mixture was stirred at the maintained temperature for 20 to 30 minutes, and allowed to stand. The layers were separated. The aqueous phase was extracted with ethyl acetate (65.49 kg), and the organic phases were combined. The combined organic phase was washed with an aqueous solution of potassium dihydrogen phosphate (prepared from potassium dihydrogen phosphate (2.96 kg) and water (16.37 kg)), then washed with an aqueous solution of sodium bicarbonate (prepared from sodium bicarbonate (5.91 kg) and water (73.45 kg)), and finally washed with an aqueous solution of sodium chloride (prepared from sodium chloride (8.41 kg) and water (33.43 kg)). Activated carbon (4.55 kg) was added. The mixture was stirred at 20 to 30 °C for 1 hour ± 10 minutes, and filtered under pressure. The filter cake was rinsed with THF (22.74 kg) and ethyl acetate (11.37 kg). The filtrates were combined, and distilled under reduced pressure until no obvious distillate appeared. Ethyl acetate (11.37 kg) was added. The mixture was distilled under reduced pressure until no obvious distillate appeared, and then distillation was continued for 1 to 2 hours. Water (2.05 kg) and ethyl acetate (102.33 kg) were added. The temperature was heated to 40 to 45 °C. The mixture was stirred. After a solid precipitated, the resultant was further maintained at the temperature and stirred for 1 hour ± 10 minutes. The temperature was slowly cooled to 0 to 5 °C. The mixture was maintained at the temperature and stirred for 1 to 2 hours. The resultant was filtered, and the filter cake was rinsed with pre-cooled ethyl acetate. Water (43.43 kg) was added. The mixture was stirred at 5 to 15 °C for 1 to 2 hours, and filtered. The filter cake was rinsed with pre-cooled water, and dried to obtain 16.62 kg of Upadacitinib hemihydrate with a purity of 99.89%, ESI-MS m/z: 390 [M+H]⁺, and no compound of formula 4 was detected.

### Example 4. Preparation of Upadacitinib hemihydrate

Ethanol (95 kg) and Upadacitinib (19.0 kg, prepared with reference to the method of Example 2-2) were added to Reaction Kettle 1, which were stirred to dissolve the reaction system. Activated carbon (0.95 kg) was added. The mixture was stirred at 25 °C to 30 °C for 2 h, and filtered. The filter residue was rinsed with ethanol (19 kg), and the filtrates were combined in Reaction Kettle 2. The filtrate was concentrated at 45 °C to constant weight, and EA (34.2 kg × 2) was added twice during the concentration process. After the distillation was completed, EA (38 kg) was added. The mixture was stirred to dissolve the solid, which was transferred to Reaction Kettle 3, Reaction Kettle 2 was washed with EA (19 kg), and the solution was transferred to Reaction Kettle 3. EA (28.5 kg) and water (0.87 kg) were added to Reaction Kettle 1. The resultant was filtered, and transferred to Reaction Kettle 2. At 40 °C to 45 °C, the solution in Reaction Kettle 3 was added dropwise to Reaction Kettle 2, and the dropwise addition time was 3 h to 4 h. After completion of the dropwise addition, the mixture was stirred at 40 °C to 45 °C for 1 h. After completion of the stirring, the temperature was cooled to 0 °C to 5 °C at a rate of 0.16 °C/min to 0.25 °C/min. The mixture was maintained at the temperature and stirred for 1 h to 2 h. The mixture was filtered. The filter cake was rinsed with EA (9.5 kg), and spin-filtered until no obvious liquid flowed out from the mother liquor outlet of the centrifuge. The wet product was dried in an oven with an oven water tank temperature of 45 °C to 55 °C and a vacuum gauge pressure of ≤-0.08 MPa, and vacuum dried for 8 h to 12 h to obtain 17.88 kg of Upadacitinib hemihydrate with a purity of 99.93%, ESI-MS m/z: 390 [M+H]+. No compound of formula 4 was detected.

### Example 5. Preparation of Upadacitinib hemihydrate

Ethanol (86.32 kg) and Upadacitinib (16.6 kg, prepared with reference to the method of Example 3-2) were added to Reaction Kettle 1, and stirred to dissolve the reaction system. Activated carbon (0.83 kg) was added at 20 to 30 °C. The mixture was stirred for 1 hour ± 10 minutes, then filtered, and rinsed with ethanol (33.2 kg). The filtrates were combined in Reaction Kettle 2, and distilled under reduced pressure until no obvious distillate appeared. Ethyl acetate (29.88 kg) was added. The mixture was distilled under reduced pressure until no obvious distillate appeared. Then, ethyl acetate (29.88 kg) was added again. The mixture was distilled under reduced pressure until no obvious distillate appeared, and then distillation was continued for 1 h to 2 h. Ethyl acetate (33.2 kg) was added, and the temperature was heated to 40 to 45 °C. The mixture was stirred to dissolve, and transferred to Reaction Kettle 3. Reaction Kettle 2 was rinsed with EA (8.3 kg). The rinsings were transferred to Reaction Kettle 3, and the temperature was maintained at 40 to 45 °C. A mixed solution of ethyl acetate (24.9 kg) and water (0.83 kg) was added to Reaction Kettle 2, to which the solution in Reaction Kettle 3 was slowly added dropwise at 40 to 45 °C. After completion of the dropwise addition, Reaction Kettle 3 was rinsed with EA. The rinsings were transferred to Reaction Kettle 2, and the mixture was maintained at the temperature and stirred for 1 hour ± 10 minutes. The temperature was cooled to 0 to 5 °C. The mixture was stirred for 1 h to 2 h, and filtered. The filter cake was rinsed with pre-cooled EA. After drying, 15.14 kg of Upadacitinib hemihydrate was obtained with a purity of 99.9%, ESI-MS m/z: 390 [M+H]⁺, and no compound of formula 4 was detected.

### Comparative Example 1-1. Synthesis of Upadacitinib hemihydrate

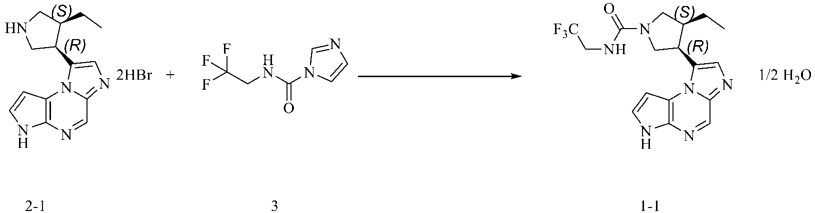

The compound of formula 2-1 (8.31 g), THF (13 mL), water (13 mL) and 20% KOH solution (11.14 g) were added to a reaction flask, and stirred to dissolve. K₂HPO₄ (6.9 g) was added to adjust the pH to 7.5 to 8.5. At 20 °C to 30 °C, the solution of the compound of formula 3 (49.0 g) in Example 1-1 was added dropwise, and the dropwise addition time was 2 h to 3 h. During the dropwise addition process, the pH of the aqueous phase was adjusted with 20% KOH solution to maintain the pH of the aqueous phase at 7.5 to 8.5. After completion of the dropwise addition, the reaction was carried out at 20 °C to 30 °C to allow the compound of formula 2-1 to react completely. During the reaction process, the pH of the aqueous phase was adjusted with 20% KOH solution to maintain it at 7.5 to 8.5. After completion of the reaction, EA (26 mL) and 20% citric acid aqueous solution (26 mL) were added to quench the reaction, and the aqueous layer was separated and removed. The organic layer was washed successively with 15% KH₂PO₄ solution (26 mL), saturated NaHCO₃ solution (26 mL), and 20% NaCl solution (13 mL), and concentrated. EA (37.4 g) and water (0.74 g) were added for crystallization to obtain 6.43 g of Upadacitinib hemihydrate with a purity of 99.74%, ESI-MS m/z: 390 [M+H]⁺, and the content of the compound of formula 4 was 0.12%.

### Comparative Example 1-2. Scale-up synthesis of Upadacitinib hemihydrate

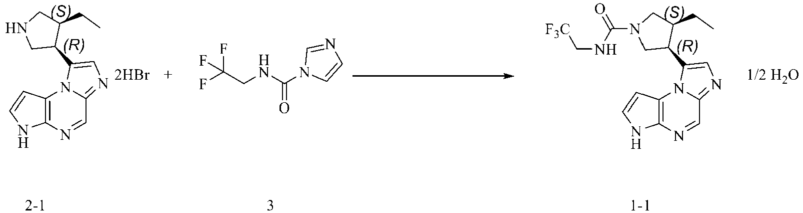

The compound of formula 2-1 (0.83 kg), THF (1.3 L), water (1.3 L) and 20% KOH solution (1.11 kg) were added to a reaction flask, and stirred to dissolve. K₂HPO₄ (0.7 kg) was added to adjust the pH to 7.5 to 8.5. At 20 °C to 30 °C, the solution of the compound of formula 3 (4.9 kg, prepared by the same method as Example 1-1, i.e., the feed amounts were: CDI 484 g, trifluoroethylamine free base 317 g, THF 2 L and 2.6 L respectively) was added dropwise, and the dropwise addition time was 2 h to 3 h. During the dropwise addition process, the pH of the aqueous phase was adjusted with 20% KOH solution to maintain the pH of the aqueous phase at 7.5 to 8.5. After completion of the dropwise addition, the reaction was carried out at 20 °C to 30 °C to allow the compound of formula 2-1 to react completely. During the reaction process, the pH of the aqueous phase was adjusted with 20% KOH solution to maintain it at 7.5 to 8.5. After completion of the reaction, EA (2.6 L) and 20% citric acid aqueous solution (2.6 L) were added to quench the reaction, and the layers were separated. The aqueous layer was extracted with EA (2.6 L), and the organic layers were combined. The organic layer was washed successively with 15% KH₂PO₄ solution (26 L), saturated NaHCO₃ solution (26 L), and 20% NaCl solution (13 L), and concentrated. EA (3.74 kg) and water (74 g) were added for crystallization to obtain 0.57 kg of Upadacitinib hemihydrate with a purity of 99.52%, ESI-MS m/z: 390 [M+H]⁺, and the content of the compound of formula 4 was 0.12%.

Among the above, in Comparative Example 1-2, the monitoring data of the purity of Upadacitinib or the hydrate thereof and the content of the compound of formula 4 during the post-treatment process are shown in Table 2:

**Table 2 Purity of Upadacitinib or the hydrate thereof and content of the compound of formula 4**

| Operation | Purity of Upadacitinib or the hydrate thereof (%) | Content of the compound of formula 4 (%) |
|---|---|---|
| After quenching with citric acid aqueous solution | 94.79 | 3.43 |
| After washing with KH₂PO₄ solution | 94.64 | 3.23 |
| After washing with NaHCO₃ | 82.35 | 14.75 |

## Claims

1. A preparation method for Upadacitinib or a hydrate thereof, wherein the method comprises the following steps:
(1) reacting a compound of formula 2 with a compound of formula 3 in an organic solvent and water;
(2) mixing a reaction system obtained from step (1) with an inorganic base A and reacting to obtain a compound of formula 1 or a hydrate thereof,

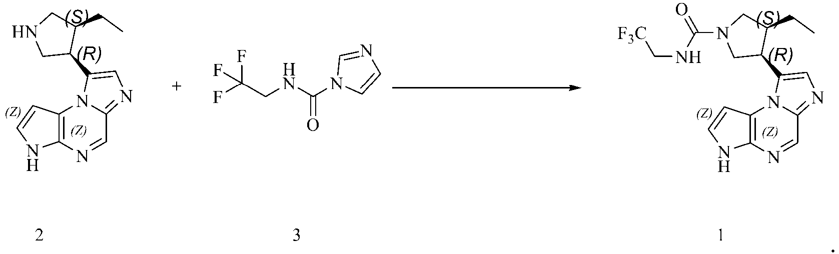

2. The preparation method according to claim 1, wherein the compound of formula 1 or the hydrate thereof is in a form of formula 1-A, 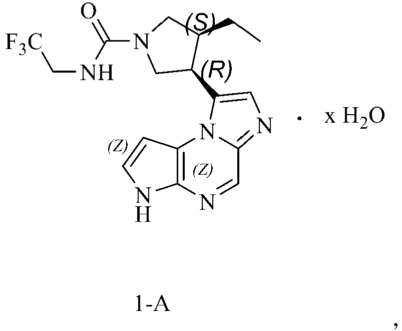 wherein x is 0 to 0.65, preferably x is 0 to 0.5, more preferably x is 0 or 0.5, still more preferably x is 0, and most preferably x is 0.5.

3. The preparation method according to claim 1 or 2, wherein,
in step (1), a molar ratio of the compound of formula 2 to the compound of formula 3 is 1:1, 1:1.3, 1:1.4, 1:1.5, 1:1.6, 1:2, 1:2.1, 1:2.2, 1:2.5, 1:3, 1:4, or a range between any two of the above ratios, preferably 1:1 to 4, more preferably 1:1 to 3, still more preferably 1:1 to 2.5, further preferably 1:1.4 to 2.2, most preferably 1:1.4 to 1.5, and most preferably 1:2 to 2.2;
in step (1), a feeding mode of the compound of formula 3 is dropwise addition, and the dropwise addition time is 0 hour to 10 hours, preferably 1 hour to 5 hours, more preferably 2 hours to 4 hours, and still more preferably 2 hours to 3 hours;
a reaction temperature in step (1) is 0 °C to 50 °C, preferably 10 °C to 40 °C, more preferably 25 °C to 35 °C, and still more preferably 20 °C to 30 °C;
in step (1), a reaction time after the addition of the compound of formula 3 is completed is 0 hour to 10 hours, preferably 0 hour to 5 hours, more preferably 1 hour to 3 hours, and still more preferably 1 hour to 2 hours;
the organic solvent in step (1) is one or more selected from the group consisting of tetrahydrofuran, 2-methyltetrahydrofuran, 1,4-dioxane, dichloromethane, and acetonitrile; preferably one or more selected from the group consisting of tetrahydrofuran, 2-methyltetrahydrofuran, and acetonitrile; more preferably one selected from the group consisting of tetrahydrofuran and 2-methyltetrahydrofuran; and still more preferably tetrahydrofuran;
in step (1), a mass-to-volume ratio (g:mL) of the compound of formula 2 to the organic solvent is 1:1, 1:8, 1:10, 1:11, 1:12, 1:16, 1:17, 1:18, 1:20, 1:22, 1:25, or a range between any two of the above ratios, preferably 1:1 to 25, more preferably 1:8 to 22, still more preferably 1:10 to 20, further preferably 1:10 to 18, most preferably 1:10 to 12, most preferably 1:11 to 18, and most preferably 1:16 to 18;
in step (1), a mass-to-volume ratio (g:mL) of the compound of formula 2 to water is 1:1 to 16, preferably 1:1 to 10, and more preferably 1:2 to 8.

4. The preparation method according to any one of claims 1 to 3, wherein,
the inorganic base A in step (2) is selected from the group consisting of sodium hydroxide, potassium hydroxide, lithium hydroxide, sodium carbonate, potassium carbonate, and lithium carbonate; preferably selected from the group consisting of sodium hydroxide, potassium hydroxide, and lithium hydroxide; more preferably sodium hydroxide or potassium hydroxide; and still more preferably potassium hydroxide;
in step (2), the inorganic base A is added in a solid form thereof or in a form of an aqueous solution thereof, preferably added in the form of an aqueous solution thereof, more preferably added in the form of an aqueous solution thereof with a mass fraction of 5% to 50%, still more preferably added in the form of an aqueous solution thereof with a mass fraction of 15% to 40%, further preferably added in the form of an aqueous solution thereof with a mass fraction of 15% to 25%, and most preferably added in the form of an aqueous solution thereof with a mass fraction of 20%;
in step (2), a molar ratio of the compound of formula 2 to the inorganic base A, calculated as monovalent basic anions of the inorganic base A, is 1:1 to 5, preferably 1:1 to 4, more preferably 1:1.5 to 2.5, still more preferably 1:2 to 2.5, further preferably 1:2 to 2.4, and most preferably 1:2.1 to 2.4;
a mixing temperature in step (2) is 0 °C to 50 °C, preferably 10 °C to 50 °C, more preferably 15 °C to 35 °C, and still more preferably 20 °C to 30 °C;
a reaction time in step (2) is 0 hour to 10 hours, preferably 1 hour to 6 hours, more preferably 2 hours to 5 hours, still more preferably 4 hours to 6 hours, and most preferably 3 hours to 4 hours;
in step (2), after mixing and reacting, the method optionally further comprises adjusting a pH value to 3 to 6, preferably further sequentially comprises adjusting the pH value to 4 to 6, extracting and performing layer separation, more preferably further sequentially comprises adjusting the pH value to 4.8 to 6, extracting and performing layer separation, still more preferably further sequentially comprises adjusting the pH value to 4.8 to 5.5, extracting with ethyl acetate and performing layer separation, and still more preferably further sequentially comprises adjusting the pH value to 5 to 6, extracting with ethyl acetate and performing layer separation.

5. The preparation method according to any one of claims 1 to 4, wherein before step (1), the method further comprises: (1a) reacting an acid addition salt of the compound of formula 2 with an inorganic base B in an organic solvent and water to obtain the compound of formula 2.

6. The preparation method according to claim 5, wherein,
the acid addition salt of the compound of formula 2 in step (1a) is selected from the group consisting of a hydrobromide, a hydrochloride, a sulfate, and a phosphate of the compound of formula 2; preferably selected from the group consisting of the hydrobromide, the hydrochloride, and the sulfate of the compound of formula 2; more preferably the hydrobromide or the hydrochloride of the compound of formula 2; and still more preferably the hydrobromide of the compound of formula 2;
the inorganic base B in step (1a) is selected from the group consisting of sodium hydroxide, potassium hydroxide, lithium hydroxide, sodium carbonate, potassium carbonate, and lithium carbonate; preferably selected from the group consisting of sodium hydroxide, potassium hydroxide, and lithium hydroxide; more preferably sodium hydroxide or potassium hydroxide; and still more preferably potassium hydroxide;
in step (1a), a molar ratio of the acid addition salt of the compound of formula 2 to the inorganic base B, calculated as monovalent basic anions of the inorganic base B, is 1:1 to 5, preferably 1:1 to 4, more preferably 1:1.5 to 2.5, still more preferably 1:1.8 to 2.2, and most preferably 1:2; and most preferably the inorganic base B is potassium hydroxide, and the molar ratio of the acid addition salt of the compound of formula 2 to the inorganic base B is 1:2;
a pH of a reaction system in step (1a) is adjusted to 8 to 9 by adding the inorganic base B;
in step (1a), the inorganic base B is added in a form of an aqueous solution thereof; preferably the inorganic base B is added in the form of an aqueous solution thereof, and the mass of the inorganic base B accounts for 20% of the mass of the aqueous solution thereof; more preferably the inorganic base B is added in the form of an aqueous solution of potassium hydroxide thereof, and the mass of potassium hydroxide accounts for 20% of the mass of the aqueous solution thereof;
the organic solvent in step (1a) is one or more selected from the group consisting of tetrahydrofuran, 2-methyltetrahydrofuran, 1,4-dioxane, dichloromethane, and acetonitrile; preferably one or more selected from the group consisting of tetrahydrofuran, 2-methyltetrahydrofuran, and acetonitrile; more preferably tetrahydrofuran or 2-methyltetrahydrofuran; and still more preferably tetrahydrofuran;
in step (1a), a mass-to-volume ratio (g:mL) of the acid addition salt of the compound of formula 2, calculated as its free base, to the organic solvent is 1:1 to 16, preferably 1:1.5 to 5, more preferably 1:2.3 to 5, still more preferably 1:2.3 to 4, and most preferably 1:2.3 to 2.6;
a reaction temperature in step (1a) is 0 °C to 50 °C, preferably 10 °C to 50 °C, more preferably 15 °C to 35 °C, and still more preferably 20 °C to 30 °C.

7. The preparation method according to any one of claims 1 to 6, wherein before step (1), the method further comprises: (1b) reacting N,N'-carbonyldiimidazole and trifluoroethylamine or a salt thereof in a solvent to obtain the compound of formula 3.

8. The preparation method according to claim 7, wherein,
a reaction temperature of the reaction in step (1b) is -10 °C to 30 °C, preferably -10 °C to 10 °C, and more preferably -5 °C to 5 °C;
the trifluoroethylamine or the salt thereof in step (1b) is trifluoroethylamine or the hydrochloride thereof, preferably trifluoroethylamine or the monohydrochloride thereof, more preferably trifluoroethylamine free base, and still more preferably trifluoroethylamine monohydrochloride;
the trifluoroethylamine or the salt thereof in step (1b) is an acid addition salt of trifluoroethylamine, and the preparation method further comprises adding a base to the reaction solution after the reaction; preferably, the trifluoroethylamine or the salt thereof in step (1b) is an acid addition salt of trifluoroethylamine, and the preparation method further comprises adding an aqueous solution of a base to the reaction solution after the reaction; more preferably, the trifluoroethylamine or the salt thereof in step (1b) is an acid addition salt of trifluoroethylamine, and the preparation method further comprises adding an aqueous solution of potassium hydroxide to the reaction solution after the reaction; still more preferably, the trifluoroethylamine or the salt thereof in step (1b) is an acid addition salt of trifluoroethylamine, and the preparation method further comprises adding a 20% aqueous solution of potassium hydroxide to the reaction solution after the reaction; and most preferably, the trifluoroethylamine or the salt thereof in step (1b) is trifluoroethylamine monohydrochloride, and the preparation method further comprises adding a 20% aqueous solution of potassium hydroxide to the reaction solution after the reaction, and the molar ratio of trifluoroethylamine monohydrochloride to potassium hydroxide is 1:1;
in step (1b), a molar ratio of the trifluoroethylamine or the salt thereof to N,N'-carbonyldiimidazole is 1:0.5, 1:0.8, 1:0.84, 1:0.9, 1:0.94, 1:0.95, 1:1, 1:1.5, 1:2, or a range between any two of the above ratios, preferably 1:0.5 to 2, more preferably 1:0.5 to 1.5, still more preferably 1:0.5 to 1, further preferably 1:0.8 to 1, most preferably 1:0.85 to 0.95, and most preferably 1:0.9 to 0.95;
the solvent for the reaction in step (1b) is one or more selected from the group consisting of tetrahydrofuran, 2-methyltetrahydrofuran, 1,4-dioxane, dichloromethane, and acetonitrile; preferably one or more selected from the group consisting of tetrahydrofuran, 2-methyltetrahydrofuran, and acetonitrile; more preferably tetrahydrofuran or 2-methyltetrahydrofuran; and still more preferably tetrahydrofuran;
in step (1b), a mass-to-volume ratio (g:mL) of N,N'-carbonyldiimidazole to the solvent is 1:1, 1:5, 1:7, 1:8, 1:9, 1:10, 1:11, 1:12, 1:14, 1:20, 1:25, or a range between any two of the above ratios, preferably 1:1 to 25, more preferably 1:5 to 20, still more preferably 1:7 to 14, further preferably 1:8 to 12, most preferably 1:7 to 10, most preferably 1:8 to 10, and most preferably 1:9 to 10;
a reaction time of the reaction in step (1b) is 0.5 hour to 3 hours, preferably 1 hour to 2 hours;
the reaction in step (1b) is carried out under a nitrogen atmosphere.

9. The preparation method according to any one of claims 5 to 8, wherein,
a molar ratio of the acid addition salt of the compound of formula 2 in step (1a) to the trifluoroethylamine or the salt thereof in step (1b) is 1:1, 1:1.5, 1:1.6, 1:1.8, 1:2, 1:2.3, 1:2.4, 1:2.5, 1:3, or a range between any two of the above ratios, preferably 1:1 to 3, more preferably 1:1 to 2.5, still more preferably 1:1 to 2, further preferably 1:1.5 to 2.5, most preferably 1:1.5 to 1.8, and most preferably 1:2.3 to 2.5;
a resultant of step (1b) is used directly in the next step without purification.

10. A preparation method for Upadacitinib hemihydrate, wherein, adding an ethyl acetate solution of crude Upadacitinib to a mixed solvent of ethyl acetate and water under 0 °C to 80 °C, after the addition, maintaining at a temperature for 1 hour to 12 hours, cooling to -10 °C to 10 °C, and crystallizing for 0 hour to 4 hours.

11. The preparation method according to claim 10, wherein,
the crude Upadacitinib is a Upadacitinib free base or a Upadacitinib hydrate, preferably a Upadacitinib free base, preferably a Upadacitinib hydrate, and more preferably a Upadacitinib hemihydrate;
a mass ratio (kg:kg) of the crude Upadacitinib to ethyl acetate in the ethyl acetate solution of crude Upadacitinib is 1:1, 1:2, 1:2.2, 1:2.5, 1:3, 1:3.2, 1:6, 1:9, 1:10, or a range between any two of the above ratios, preferably 1:1 to 10, more preferably 1:2 to 9, still more preferably 1:2 to 6, further preferably 1:2.2 to 3.2, and most preferably 1:2.5 to 3;
a molar ratio of the crude Upadacitinib to water is 1:0.5 to 4, preferably 1:0.5 to 2, more preferably 1:0.8 to 2, and still more preferably 1:0.8 to 1.2;
a total mass of ethyl acetate refers to the total amount of ethyl acetate used in the ethyl acetate solution of crude Upadacitinib and the mixed solvent of ethyl acetate and water in the preparation method, and the mass ratio of water to the total mass of ethyl acetate is 1:200 to 1:32, preferably 1:200 to 1:50, more preferably 1:100 to 1:50, and still more preferably 1:100 to 1:78;
the preparation method further comprises that a dropwise addition time of the ethyl acetate solution of crude Upadacitinib is 1 hour to 6 hours, preferably 1 hour to 5 hours, more preferably 2 hours to 5 hours, and still more preferably 3 hours to 4 hours;
the maintained temperature is 0 °C to 50 °C, preferably 20 °C to 50 °C, more preferably 30 °C to 45 °C, and still more preferably 40 °C to 45 °C;
the period of time at the maintained temperature is 1 hour to 10 hours, preferably 2 hours to 8 hours, more preferably 3 hours to 6 hours, and still more preferably 4 hours to 5 hours;
the temperature after cooling is -5 °C to 10 °C, preferably -5 °C to 5 °C, and more preferably 0 °C to 5 °C;
a cooling rate is 0.15 °C/min to 1.0 °C/min, preferably 0.15 °C/min to 0.75 °C/min, more preferably 0.15 °C/min to 0.5 °C/min, and still more preferably 0.15 °C/min to 0.3 °C/min;
the crystallization time is 0 hour to 3 hours, preferably 0.5 hour to 3 hours, and more preferably 0.5 hour to 2 hours.

12. A high-purity Upadacitinib hemihydrate, wherein a content of a compound of formula 4 therein is less than 0.1%, and a purity of the Upadacitinib hemihydrate is higher than 98.0%,

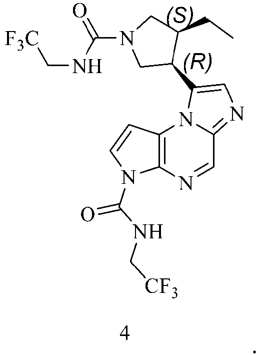

13. The Upadacitinib hemihydrate according to claim 12, wherein,
the content of the compound of formula 4 is less than 0.05%, preferably less than 0.03%, and more preferably less than 0.015%;
the purity of the Upadacitinib hemihydrate is higher than 98.5%, preferably higher than 99.0%, more preferably higher than 99.5%, and still more preferably higher than 99.8%.
